# FASCICULE DE BREVET EUROPEEN

(11) **EP 0 445 041 B1**
(45) Date de publication et mention de la délivrance du brevet: **03.04.1996**
(21) Numéro de dépôt: 91400572.3
(22) Date de dépôt: 01.03.1991
(51) Int. Cl.: B01D 53/22, C07C 7/00

(54) **Procédé de séparation de mélanges gazeux obtenus dans les raffineries de pétrole**
Verfahren zur Trennung von Gasgemischen aus Erdölraffinerien
Process for separating gas mixings from petroleum refineries

(30) Priorité: 02.03.1990 FR 9002645
(43) Date de publication de la demande: 04.09.1991
(73) Titulaire: SOCIETE POUR L' EQUIPEMENT DES INDUSTRIES CHIMIQUES, F-95864 Cergy Pontoise Cédex (FR)
(72) Inventeur: Cicile, Jean-Charles, F-75019 Paris (FR); Cros, Pierre, F-75016 Paris (FR); Tassart, Michel, F-75018 Paris (FR)
(74) Mandataire: Keib, Gérard

(56) Documents cités:
- EP-A- 0 178 172
- EP-A- 0 359 628
- DE-C- 3 315 930

## Description

La présente invention concerne un procédé de séparation de mélanges gazeux obtenus dans les raffineries de pétrole et les usines pétrochimiques contenant du méthane, des hydrocarbures à deux, trois, quatre et cinq atomes de carbone et éventuellement de quantités notables d'hydrogène.

L'invention vise en particulier le traitement des mélanges gazeux suivants :
- ceux formés en haut de la colonne de distillation atmosphérique d'une raffinerie de pétrole brut,
- ceux issus des unités de reformage catalytique,
- ceux formés dans les unités d'hydro-traitement du naphta et du gasole, destinées à éliminer le soufre,
- ceux formés dans les unités d'hydro-traitement des essences,
- ceux formés dans les unités d'hydro-désulfuration autres que celles ci-dessus, et
- ceux formés dans les unités de craquage catalytique.

Les gaz formés dans les unités ci-dessus renferment, en des proportions variables, du méthane, des hydrocarbures à deux, trois, quatre et cinq atomes de carbone (C₂, C₃, C₄, C₅) et éventuellement des quantités notables d'hydrogène.

Ces mélanges gazeux sont souvent utilisés comme combustible dans les raffineries de pétrole pour les divers besoins de chauffage. Ils peuvent également être exportés ailleurs au moyen de canalisations.

Ces mélanges gazeux ne sont actuellement pas stockés, de sorte que leur valeur commerciale est celle du combustible interne, qui peut être quasiment nulle.

Les demanderesses se sont intéressées au problème de la séparation de ces mélanges gazeux en diverses fractions susceptibles d'être commercialisables et de les valoriser ainsi d'une manière significative.

L'état de la technique en ce qui concerne la séparation des gaz de faible masse molaire utilise actuellement la distillation cryogénique, c'est-à-dire à très basse température.

Ainsi, la technique de séparation par distillation cryogénique, qui est utilisée dans la production des gaz de l'air vers - 170°C et à pression inférieure à 5 bars, a été appliquée à basse pression (< 10 bars) et à une température d'environ - 70°C pour la récupération des hydrocarbures en C₃ - C₄ - C₅ et à - 170°C pour la récupération de l'hydrogène à haute pureté.

Ces applications amènent à transposer à des gaz qui contiennent de nombreuses impuretées (H₂O, H₂S, CO₂, hydrocarbures en C₅ et plus) une technique cryogénique conçue pour des gaz moins chargés d'impuretés (l'air) et de composition stable. En effet, les gaz de raffinerie sont par essence de composition variable en raison de la diversité des pétroles bruts traités et de la variation des paramètres de réglage des unités.

Toutefois, l'utilisation de ces techniques de séparation par cryogénie est onéreuse en raison des très basses températures mises en oeuvre. Cette technique n'est par conséquent pas indiquée dans le cas d'une séparation d'un mélange gazeux dont la valeur économique est celle du combustible interne,qui peut être quasiment nulle.

Le but de la présente invention est ainsi de proposer un procédé économique de séparation de mélanges gazeux obtenus dans les raffineries de pétrole, renfermant du méthane et des hydrocarbures à deux, trois, quatre et cinq atomes de carbone qui permet de valoriser ce mélange gazeux d'une manière très substantielle.

Suivant l'invention, ce procédé est caractérisé par les étapes selon la revendication 1.

Les revendications 2 à 9 définissent des modes de réalisation préférés. En particulier, la revendication 2 concerne un procédé par un mélange gazeux contenant de l'hydrogène.

Le procédé selon l'invention permit ainsi de séparer le mélange gazeux de départ, en trois fractions, à savoir :
- une fraction constituée principalement de méthane pouvant être utilisée comme substitut de gaz naturel pour les besoins de chauffage, notamment domestique; cette fraction est appelée GNS (gaz naturel substituable),
- une fraction renfermant des hydrocarbures en C₂, en particulier de l'éthylène dont la valeur économique est également très intéressante,
- une fraction contenant principalement des hydrocarbures en C₃ et C₄ (propane-butane). Cette fraction est commercialisable en tant que GPL (gaz de pétrole liquéfié) pouvant directement servir de combustible pour certains véhicules notamment des automobiles si la teneur du gaz traité en hydrocarbures à cinq atomes de carbone est faible.

Dans le cas d'un mélange gazeux contenant de l'hydrogène et d'un procédé avec perméation gazeuse, on récupére une quatrième fraction constitueé principalement d'hydrogène.

Les quatre fractions ci-dessus, au lieu de constituer un gaz fatal et concurrent du combustible (fioul) liquide, ont des débouchés à valeur ajoutée notablement supérieure, soit à l'intérieur (gaz riche en hydrogène) soit à l'extérieur de la raffinerie (GNS, coupe C₂, GPL). Ainsi cette valorisation du gaz est équivalente à une conversion de fioul lourd.

Le procédé selon l'invention, ne nécessite que des refroidissements modérés (- 20°C), ce qui le rend économique et parfaitement compatible avec l'objectif de valorisation recherché.

Selon une version avantageuse de l'invention, lors de la première étape, on comprime le mélange gazeux à une pression comprise entre 40 et 85 bars.

Ces valeurs de pression peuvent être obtenues facilement au moyen de compresseurs usuels.

Selon une version préférée de l'invention, l'étape de condensation partielle est précédée d'une étape d'épuration pour éliminer H₂O, H₂S et CO₂.

Cette étape d'épuration peut être effectuée à l'aide de moyens couramment mis en oeuvre dans les raffineries de pétrole.

Selon une autre version avantageuse du procédé, l'étape de distillation est réalisée sous une pression sensiblement comprise entre 25 et 38 bars.

Selon une autre version du procédé, l'étape de distillation est réalisée à l'aide de plusieurs colonnes à distiller.

Selon une autre version du procédé, la fraction gazeuse non condensée au cours de l'étape de condensation est envoyée dans une colonne d'adsorption de façon à améliorer le rendement de récupération des hydrocarbures à deux atomes de carbone.

La mise en oeuvre de l'étape de perméation gazeuse peut être réalisée selon une technique bien connue, notamment décrite dans le brevet américain 4 654 047.

D'autres particularités et avantages de l'invention apparaîtront encore dans la description ci-après d'un procédé avec perméation gazeuse.

Aux dessins annexés donnés à titre d'exemples non limitatifs :
- la figure 1 illustre schématiquement un premier mode de réalisation d'une installation de séparation de gaz conforme à l'invention,
- la figure 2 illustre schématiquement un second mode de réalisation de l'installation.

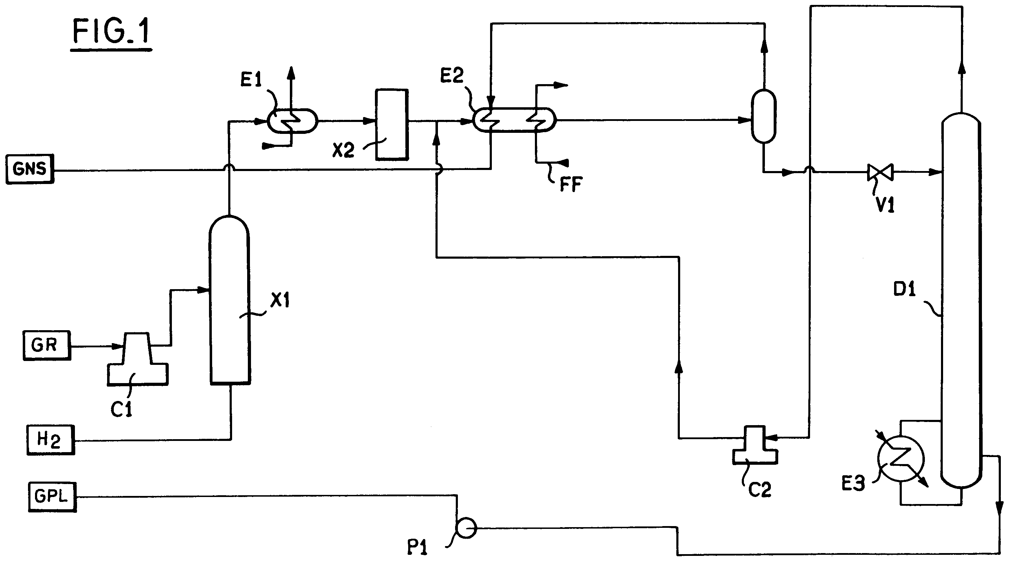

La figure 1 montre une installation permettant de produire du GNS (gaz naturel substituable) à 70 bars, du GPL (gaz de pétrole liquéfiable) et un gaz riche en hydrogène (H₂).

Le gaz de raffinerie (GR) est comprimé dans le compresseur C₁ à une pression suffisante pour qu'il ne soit pas nécessaire de recomprimer le gaz substituable obtenu finalement, c'est-à-dire à une pression comprise entre 75 et 85 bars. Le gaz comprimé passe dans le séparateur à membranes X₁ qui sépare, par perméation, un flux riche en hydrogène (H₂) qui est passé à travers les membranes et qui est donc disponible à basse pression. Le gaz résiduaire, enrichi en hydrocarbures, est refroidi dans l'échangeur de chaleur E₁ et séché sur les tamis moléculaires X₂ de façon à éviter la formation d'hydrates dans l'installation. Le gaz séché est refroidi dans l'échangeur de chaleur E₂ d'abord par le GNS produit qui se réchauffe avant d'être envoyé dans le réseau des utilisateurs puis par un fluide frigorifique (FF). La température à la sortie de l'échangeur E₂ est telle qu'il se produit une condensation partielle des hydrocarbures. Cette température est de préférence de l'ordre de - 20°C. Les hydrocarbures non condensés constituent le GNS. Les condensats sont détendus par passage à travers une vanne V₁ et entrent dans la colonne de distillation D₁ fonctionnant sous une pression comprise entre 25 et 38 bars et à une température en tête de l'ordre de - 15°C. Cette colonne, chauffée par le bouilleur E₃, sépare les condensats en deux fractions:
- un gaz de pétrole liquéfié (GPL) qui peut être envoyé vers une unité de fractionnement de gaz par la pompe P₁,
- un flux gazeux sortant en tête de colonne D₁ qui est recomprimé par le compresseur C₂ à une pression de l'ordre de 75 à 85 bars et recyclé avec le gaz séché en amont de l'échangeur de chaleur E₂.

Les performances de cette installation sont les suivantes :
Le traitement de 11 000 Nm3/h (soit environ 7900 kg/h) d'un gaz ayant la composition suivante (en pourcentage volumique) :

| | | | |
|---|---|---|---|
| Hydrogène | 41 | Ethane | 12 |
| Azote | 5 | Propylène | 2 |
| Méthane | 27 | Propane | 5 |
| Ethylène | 5 | Butane et plus lourds | 2 |

disponible à 15 bars, 30°C et saturé en eau, permet d'obtenir les produits suivants :
- un gaz de substitution au gaz naturel à 70 bars avec un pouvoir calorifique (PCS) de 44 726 KJ/Nm³ et un débit de 4 900 Nm3/h,
- un gaz riche en hydrogène (plus de 80% en volume) à 5 bars avec un débit de 5 000 Nm3/h,
- une coupe d'hydrocarbures liquides (GPL) sans méthane avec un débit de 1 850 kg/h.

Les consommations correspondant à cette production sont les suivantes :

| | |
|---|---|
| - Electricité | 900 kWh/h |
| - Condensats (120 à 80°C) | 4 m3/h |
| - Eau de refroidissement | 130 m3/h. |

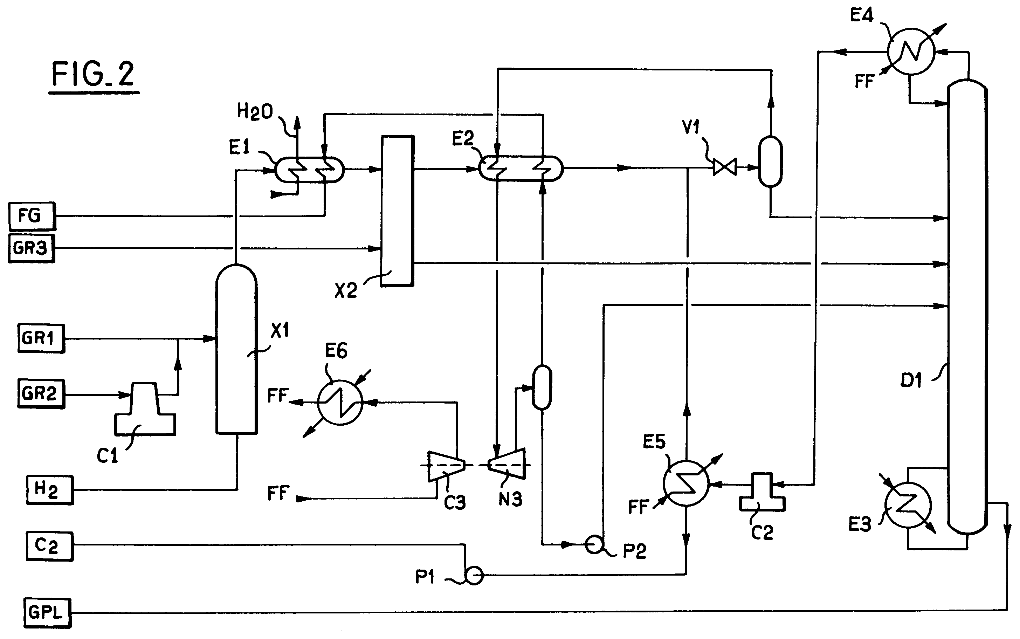

L'installation représentée sur la figure 2 permet de produire une fraction d'hydrocarbures en C₂ liquide, un GPL et un gaz riche en hydrogène.

Cette installation permet de traiter les trois gaz typiques des raffineries suivants :
- un gaz de purge de l'hydrodésulfuration à 50 bars contenant 76% d'hydrogène. Ce gaz (GR1) est envoyé directement à l'unité de perméation X₁,
- un gaz de cracking catalytique à 13 bars contenant 23% d'hydrogène. Ce gaz (GR2) est comprimé avant d'être introduit dans l'unité de perméation,
- un gaz provenant des distillations, sans hydrogène, à 32 bars. Ce gaz (GR3) est envoyé directement à l'unité de séchage X₂.

L'hydrogène (H₂) sortant de l'unité de perméation X₁ est livré en limite de batterie.

Le gaz riche en hydrocarbures sortant de l'unité de perméation X₁ est refroidi dans l'échangeur de chaleur E₁ par de l'eau (H₂O) puis par le gaz combustible FG sortant de l'installation. Après séchage dans le sécheur X₂, le gaz riche en hydrocarbures est encore refroidi dans l'échangeur de chaleur E₂ par le gaz combustible FG, puis il est détendu dans la vanne V₁ à pression comprise entre 30 et 40 bars. Les hydrocarbures restant en phase gazeuse sont réchauffés dans l'échangeur de chaleur E₂ puis détendus dans la turbine N₃. Les gaz obtenus après détente constituent la fraction fioul gaz (FG). Avant d'être remis en limite d'unité, ils sont utilisés pour refroidir le gaz à traiter (dans les échangeurs de chaleur E₂ et E₁).

La colonne de distillation D₁ est alimentée d'une part par les condensats issus de la condensation des hydrocarbures dans l'échangeur E₂ et produits au cours de la détente dans la turbine N₃, d'autre part par le gaz provenant des colonnes de fractionnement de gaz de la raffinerie (GR3). La colonne munie d'un condenseur (E₄) en haut et d'un rebouilleur (E₃) en bas sépare l'alimentation en deux produits :
- un gaz liquéfié (GPL) en fond de colonne,
- un flux gazeux en tête riche en éthane et éthylène (C₂) qui constitue le distillat de la colonne.

Ce distillat est recomprimé dans le compresseur C₂ et partiellement condensé dans l'échangeur de chaleur E₅.

Les condensats constituent la fraction C₂ qui est envoyée en limite d'unité par la pompe P1.

Les hydrocarbures non condensés sont recyclés avec l'alimentation de la colonne.

L'énergie dégagée par la détente du fioul gaz est utilisée dans le compresseur C₃ qui est le compresseur d'une boucle frigorifique fonctionnant entre le condenseur E₆ et les échangeurs de chaleur E₄ et E₅.

Les performances de cette installation sont les suivantes :
Le traitement de :
- 1 900 Nm3/h de gaz de purge à haute pression,
- 12 400 Nm3/h de gaz de cracking catalytique,
- 1 200 Nm3/h de gaz du gas plant,
permet d'obtenir :
- un gaz riche en hydrogène (plus de 80% en volume) avec un débit de 3 650 Nm3/h,
- une coupe d'hydrocarbures en C₂ liquide à 50 bars et 0°C avec un débit de 2 000 Kg/h,
- un gaz de pétrole liquifié (contenant moins de 2% d'hydrocarbures en C₂ par rapport aux hydrocarbures en C₃), à 17 bars avec un débit de 1 700 kg/h,
- un gaz combustible résiduaire à 3 bars avec un débit de 9 500 Nm3/h.

Les consommations nécessaires pour cette production sont les suivantes :

| | |
|---|---|
| - Electricité | 780 kWh/h |
| - Vapeur basse pression | 1 250 kg/h |
| - Eau de refroidissement | 100 m3/h. |

Bien entendu, l'invention n'est pas limitée aux exemples de réalisation que l'on vient de décrire.

Pour valoriser un mélange de gaz de raffinerie dépourvu d'hydrogène, il suffit d'introduire ce mélange gazeux dans l'installation de séparation en aval de l'unité de perméation gazeuse.

La production d'un GNS (gaz naturel substituable) à 70 bars environ nécessite un poste de réfrigération auxiliaire.

Dans le cas où il n'y a pas production de GNS à 70 bars environ, la détente du gaz riche en méthane assure l'autonomie énergétique de l'installation et évite l'installation d'un poste de réfrigération auxiliaire.

## Revendications

1. Procédé de séparation de mélanges gazeux (GR) obtenus dans les raffineries de pétrole, renfermant du méthane et des hydrocarbures à deux, trois, quatre et cinq atomes de carbone, ce procédé étant caractérisé par les étapes successives suivantes :
- une étape de compression (C₁) du mélange gazeux à une pression au moins égale à 30 bars,
- une étape de condensation partielle (E₂) à une température supérieure à - 22°C du mélange gazeux et d'extraction de la phase (GNS) non condensée constituée principalement de méthane ;
- une étape de distillation (D₁) à une température supérieure à - 15°C et une pression supérieure à 20 bars, de la phase condensée issue de l'étape précédente pour séparer les hydrocarbures à deux atomes de carbone, des hydrocarbures ayant trois, quatre et cinq atomes de carbones (GPL).

2. Procédé conforme à la revendication 1, le mélange gazeux renfermant en outre de l'hydrogène caractérisé en ce qu'entre l'étape de compression (C₁) et l'étape de condensation partielle (E₂), on procède à une étape de passage du mélange gazeux dans une unité de perméation gazeuse (X₁) et d'extraction d'une fraction constituée principalement d'hydrogène ayant traversé la membrane perméable de ladite unité, la fraction gazeuse n'ayant pas traversé la membrane étant soumise aux étapes suivantes du procédé.

3. Procédé conforme à la revendication 2, dans lequel, lors de la première étape, on comprime le mélange gazeux à une pression comprise entre 40 et 85 bars.

4. Procédé conforme à l'une des revendications 2 ou 3, dans lequel l'étape de condensation partielle est précédée d'une étape d'épuration (X₂) pour éliminer H₂O, H₂S et CO₂.

5. Procédé conforme à l'une des revendications 1 à 4, dans lequel l'étape de distillation est réalisée sous une pression sensiblement comprise entre 25 et 38 bars.

6. Procédé conforme à l'une des revendications 1 à 5, dans lequel l'étape de distillation est réalisée à l'aide de plusieurs colonnes en série

7. Procédé conforme à l'une des revendications 1 à 5, dans lequel la fraction gazeuse non condensée est envoyée dans un ensemble P.S.A. (traitement par absorption) pour en extraire les hydrocarbures à deux atomes de carbone.

8. Procédé conforme à l'une des revendications 1 à 6, caractérisé en ce que ladite phase condensée est détendue d'une pression de l'ordre de 75 à 85 bars jusqu'à une pression de l'ordre de 25 à 38 bars avant l'étape de distillation.

9. Procédé conforme à l'une des revendications 6 ou 7, dans lequel la phase gazeuse sortant de la tête de la colonne de distillation (D₁) est partiellement recyclée en amont de l'échangeur thermique à l'aide d'un compresseur.

## Claims

1. A method for the separation of gas mixtures (GR) obtained in oil refineries, containing methane and hydrocarbons with two, three, four, and five carbon atoms, this method being characterised in that it comprises the following five successive steps:
- a compression step (C1) wherein the gas mixture is compressed at a pressure of at least 30 bars,
- a partial condensation step (E2) wherein the gas mixture is partially condensed at a temperature above - 22°C, followed by the extraction of the non-condensed Phase (GNS) mainly consisting of methane;
- a distillation step (D1) wherein the condensed phase resulting from the previous step is exposed to a temperature above -15°C and a pressure above 20 bars in order to separate the hydrocarbons with two carbon atoms from the hydrocarbons having three, four, and five carbon atoms (GPL).

2. A method according to claim 1, with the gas mixture further containing hydrogen, characterised in that between the compression step (C1) and the partial condensation step (E2) there is an additional step wherein the gas mixture passes through a gas permeation unit (X1) and a fraction mainly consisting of hydrogen which has passed through the permeable membrane of said unit is extracted, the gas fraction which has not passed through the membrane being submitted to the next steps of the method.

3. A method according to claim 2, wherein, during the first step, the gas mixture is compressed at a pressure of between 40 and 85 bars.

4. A method according to one of claims 2 or 3, wherein the partial condensation step is preceded by a filtering step (X2) so as to eliminate H₂0, H₂S and CO₂.

5. A method according to one of claims 1 to 4, wherein the distillation step is carried out at a pressure of substantially 25 to 38 bars.

6. A method according to one of claims 1 to 5, wherein the distillation step is carried out using several columns in series.

7. A method according to one of claims 1 to 5, wherein the non-condensed gas fraction is transferred to a P.S.A. unit (absorption treatment) so that extract the hydrocarbons with two carbon atoms are extracted therefrom.

8. A method according to one of claims 1 to 6, characterised in that the pressure applied to said condensed phase is reduced from approximately 75 to 85 bars to approximately 25 to 38 bars before the distillation step.

9. A method according to one of claims 6 or 7, wherein the gas phase exiting from the head of the distillation column (D1) is partially recycled upstream from the heat exchanger by means of a compressor.

## Patentansprüche

1. Verfahren zum Trennen von in Ölraffinerien anfallenden Gasgemischen (GR), die Methan und Kohlenwasserstoffe mit zwei, drei, vier und fünf Kohlenstoffatomen enthalten, wobei dieses Verfahren durch folgende aufeinanderfolgende Schritte gekennzeichnet ist :
- einen Verdichtungsschritt (C₁) des Gasgemischs bei einem Druck von mindestens 30 bar,
- einen Teilkondensationsschritt (E₂) des Gasgemischs bei einer Temperatur von über - 22°C und einen Extraktionsschritt der nicht kondensierten, hautpsächlich aus Methan bestehenden Phase (GNS),
- einen Distillationsschritt (D₁) der kondensierten, aus dem vorhergehenden Schritt hervorgegangenen Phase bei einer Temperatur von über - 15°C und einem Druck von über 20 bar zum Trennen der Kohlenwasserstoffe mit zwei Kohlenstoffatomen von den Kohlenwasserstoffen mit drei, vier und fünf Kohlenstoffatomen (GPL).

2. Verfahren nach Anspruch 1, wobei das Gasgemisch außerdem Wasserstoff enthält, dadurch gekennzeichnet, daß zwischen dem Verdichtungsschritt ((C₁) und dem Teilkondensationsschritt (E₂) ein Überführungsschritt des Gasgemischs in eine Gaspermeationseinheit (X₁) und ein Extraktionsschritt einer hauptsächlich aus Wasserstoff bestehenden Fraktion durchgeführt wird, welche die durchlässige Membran der Einheit durchdrungen hat, wobei die Gasfraktion, die die Membran nicht durchdrungen hat, den nachfolgenden Verfahrensschritten ausgesetzt wird.

3. Verfahren nach Anspruch 2, bei dem das Gasgemisch beim ersten Schritt bei einem Druck von 40 bis 85 bar verdichtet wird.

4. Verfahren nach einem der Ansprüche 2 oder 3, bei dem vor dem Teilkondensationsschritt ein Reinigungsschritt (X₂) zum Abscheiden von H₂O, H₂S und CO₂ stattfindet.

5. Verfahren nach einem der Ansprüche 1 bis 4, bei dem der Distillationsschritt bei einem Druck von etwa 25 bis 38 bar durchgeführt wird.

6. Verfahren nach einem der Ansprüche 1 bis 5, bei dem der Distillationsschritt mit Hilfe mehrerer hintereinander angeordneter Kolonnen durchgeführt wird.

7. Verfahren nach einem der Ansprüche 1 bis 5, bei dem die nicht kondensierte Gasfraktion zum Extrahieren der darin enthaltenen Kohlenwasserstoffe mit zwei Kohlenstoffatomen in ein PSA-System (Absorptionsbehandlung) befördert wird.

8. Verfahren nach einem der Ansprüche 1 bis 6, dadurch gekennzeichnet, daß der Druck der kondensierten Phase vor dem Distillationsschritt von einem Druck der Größenordnung von 75 bis 85 bar bis auf einen Druck der Größenordnung von 25 bis 38 bar reduziert wird.

9. Verfahren nach einem der Ansprüche 6 oder 7, bei dem die aus dem Kopf der Distillationskolonne (D₁) austretende Gasphase oberhalb des Wärmeaustauschers mit Hilfe eines Kompressors teilweise zurückgeführt wird.
